(19) Europäisches Patentamt / European Patent Office / Office européen des brevets

(11) **EP 4 501 316 A1**

(12) **EUROPEAN PATENT APPLICATION**
published in accordance with Art. 153(4) EPC

(43) Date of publication:
**05.02.2025 Bulletin 2025/06**

(21) Application number: 23773671.5

(22) Date of filing: **15.03.2023**

(51) International Patent Classification (IPC):
$A61K\ 9/127^{(2025.01)}$    $A61K\ 31/7068^{(2006.01)}$
$A61K\ 47/24^{(2006.01)}$    $A61K\ 47/28^{(2006.01)}$
$A61K\ 47/02^{(2006.01)}$    $A61P\ 35/00^{(2006.01)}$

(52) Cooperative Patent Classification (CPC):
A61K 9/127; A61K 31/7068; A61K 47/02;
A61K 47/24; A61K 47/28; A61P 35/00

(86) International application number:
**PCT/CN2023/081594**

(87) International publication number:
**WO 2023/179423 (28.09.2023 Gazette 2023/39)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **25.03.2022 CN 202210300041**

(71) Applicants:
• **Sichuan Kelun Pharmaceutical Research Institute Co., Ltd.**
  **Chengdu, Sichuan 611138 (CN)**
• **Hunan Kelun Pharmaceutical Research Co., Ltd.**
  **Yueyang, Hunan 414000 (CN)**

(72) Inventors:
• **HAO, Fei**
  **Chengdu, Sichuan 611138 (CN)**

• **SU, Zhengxing**
  **Chengdu, Sichuan 611138 (CN)**
• **LI, Ming**
  **Chengdu, Sichuan 611138 (CN)**
• **ZHAO, Dong**
  **Chengdu, Sichuan 611138 (CN)**
• **LIU, Sichuan**
  **Chengdu, Sichuan 611138 (CN)**
• **ZHANG, Ruixia**
  **Chengdu, Sichuan 611138 (CN)**
• **LIU, Xia**
  **Chengdu, Sichuan 611138 (CN)**
• **JIAO, Jian**
  **Chengdu, Sichuan 611138 (CN)**

(74) Representative: **Cabinet Nony**
   **11 rue Saint-Georges**
   **75009 Paris (FR)**

(54) **GEMCITABINE LIPOSOME PHARMACEUTICAL COMPOSITION, PREPARATION METHOD THEREFOR AND USE THEREOF**

(57)    A gemcitabine liposome pharmaceutical composition, a preparation method therefor and use thereof. According to the preparation method for the liposome, by means of optimization and control of the proportion of phospholipid to cholesterol, the proportion of the total lipid to an optional long circulating functional material, the proportion of the total lipid to a drug, and the type and the concentration of a salt solution in an aqueous interior, a liposome, which has high encapsulation efficiency, good storage stability, and high filtering performance, is not easy to leak, is stable and controllable to release, prolongs the half-life remarkably, improves the therapeutic effect, and reduces toxic and side effects, is prepared. In addition, the provided liposome prescription and preparation process are simple, and scaled-up production and clinical popularization and application are facilitated.

EP 4 501 316 A1

## Description

The present application is based on the present application with CN application number 202210300041.7 and the filing date of March 25, 2022, and claims its priority. The disclosure content of the CN application is hereby incorporated into the present application in its entirety.

## Technical Field

The present invention relates to the field of pharmaceutical preparations, and specifically relates to a liposome composition of gemcitabine or pharmaceutically acceptable salts thereof and its preparation method and uses.

## Background Art

Gemcitabine is a metabolic anti-cancer drug with wide clinical applications. It is used as first-line chemotherapy regimen for a variety of solid tumors (including pancreatic cancer, breast cancer, non-small cell lung cancer, ovarian cancer, etc.) alone or in combination with other drugs. Due to its simple molecular structure and strong hydrophilicity, it is rapidly metabolized and excreted by the liver and kidneys after entering the body, resulting in its extremely short half-life, poor targeting, and high clinical dose, which often cause severe blood system toxicity, thereby limiting its clinical use.

As nanoscale drug carriers, liposomes have the advantages of safety, nontoxicity, good biocompatibility, sustained release, and targeting to tumor tissues/cells through high permeability and retention effect (EPR). After a drug is encapsulated into liposomes, the rapid clearance of the drug can be avoided, the blood circulation time can be prolonged, the targeting to tumor tissues/cells can be enhanced, and the normal tissue distribution can be reduced, thereby achieving the purpose of reducing toxicity and increasing efficacy.

No liposomal formulations of gemcitabine are available on the market to date. Current research on gemcitabine liposomes faces the problems of fast release, instability, low encapsulation efficiency, complex processes, difficulty in industrial scale-up production, easy leakage in the body, and high safety risks; at the same time, it also faces the shortcomings of insufficient research on the types and formulation ratios of drugs and phospholipid excipients and buffer salt systems. CN106474067B discloses a polyethylene glycol-modified gemcitabine liposome, which has a low encapsulation efficiency (about 70%) and poor stability (approximately 30 days of stability), making it difficult for industrial production and clinical application. CN102784107B discloses a gemcitabine liposome that contains an injection oil, of which encapsulation efficiency is improved, but it requires freeze-drying to maintain its stability, resulting complex process and high cost, and thus it is not conducive to industrial production. CN102846547B discloses an emulsifier-containing gemcitabine liposome, which stability is improved, but it contains polyoxyethylene ricinoleate, resulting high safety risk and prone to causing acute hypersensitivity reaction, neurotoxicity, etc. Some documents (e.g., CN111035616A, CN111437259A, CN108348538A, CN111632030A) record the compositions and preparation methods of gemcitabine liposomes, and individual protocols thereof show that some indicators are better. However, there are still insufficient information on the studying of key parameters (e.g., selection of excipients composing liposomes, ratio of excipients, ratio of excipients to drugs, control of aqueous interior environment of liposomes, etc.) related to the stability, encapsulation efficiency, release, filterability, attenuation and synergy of liposomes.

In summary, it is of great significance to prepare a gemcitabine liposome with stable and controllable release, less leakage during storage, high encapsulation efficiency, high stability and high drug loading capacity, which at the same time can significantly prolong the blood half-life of gemcitabine, improve efficacy, and reduce toxic and side effects, and having simple composition which is feasible for industrial production, thereby meeting the clinical needs.

## Contents of the present invention

The purpose of the present application is to provide a liposome of gemcitabine or pharmaceutically acceptable salts thereof, which has high drug loading capacity, high encapsulation efficiency, good storage stability and filterability, stable and controllable release, feasible for industrial scale-up production, lower toxic and side effects and/or better drug efficacy. Compared with gemcitabine conventional injection, the liposome of the present application can avoid rapid metabolism of gemcitabine or pharmaceutically acceptable salts thereof in the body, prolong blood circulation time, improve tumor targeting, improve therapeutic effect, and reduce serious hematological toxicity of the conventional injection, thereby meeting the needs of clinical improvement.

In one aspect, the present invention relates to a gemcitabine liposome, which comprises a liposome membrane and an aqueous interior encapsulated by the liposome membrane, wherein,

the components constituting the liposome membrane comprise a phospholipid, cholesterol and optionally a long-circulating functional material; the aqueous interior comprises gemcitabine or a pharmaceutically acceptable salt thereof, and a salt solution; and,

the weight ratio of the phospholipid to cholesterol is 2:1 to 20:1;

the long-circulating functional material accounts for 0% to 30% by mass of total lipids;

the weight ratio of total lipids to gemcitabine or the pharmaceutically acceptable salt thereof in the liposome is 1:1 to 100:1;

the molar concentration of the salt solution is 50 to 800 mM.

In the liposome of the present invention, the aqueous interior contains a salt solution, and the molar concentration of the salt solution refers to the molar concentration of the salt molecules in the salt solution; when the aqueous interior contains various salts, the molar concentration refers to the total molar concentration of various salts molecules in the salt solution.

In the liposome, interactions are observed between gemcitabine and lipids (including phospholipid, cholesterol, and long-circulating functional material), between gemcitabine and the salt solution of the aqueous interior, between lipids and components contained in aqueous interior environment, and among lipids, thereby forming a liposome with good stability, high encapsulation efficiency, uniform particle size, stable and controllable release, and good filterability, and the liposome can significantly prolong the circulation time of gemcitabine in the body, improve the efficacy, and reduce the toxic and side effects.

In some embodiments, the weight ratio of the phospholipid to cholesterol is 2:1 to 15:1, preferably 2:1 to 10:1, preferably 2.5:1 to 10:1, more preferably 2:1 to 8:1, such as 2:1 to 3:1 or 7:1 to 8:1.

In some embodiments, the long-circulating functional material accounts for 0% to 20%, preferably 0% to 15%, such as 0% or 0.1% to 15%, for another example 0.1% to 0.5%, 0.1% to 5%, 0.1% to 10%, 0.1% to 12%, 0.1% to 15%, 0.5% to 5%, 0.5% to 10%, 0.5% to 12%, 0.5% to 15%, 5% to 10%, 5% to 12%, 5% to 15%, 10% to 12%, 10% to 15%, or 12% to 15% by mass of total lipids (including phospholipids, cholesterol and optional long-circulating functional materials).

In some embodiments, the weight ratio of total lipids (including phospholipid, cholesterol and optional long-circulating functional material) to gemcitabine or the pharmaceutically acceptable salt thereof is 5:1 to 80:1, preferably 10:1 to 50:1, more preferably 10:1 to 40:1, such as 10:1 to 15:1, 10:1 to 20:1, 10:1 to 25:1, 10:1 to 30:1, 10:1 to 35:1, 10:1 to 40:1, 15:1 to 20:1, 15:1 to 25:1, 15:1 to 30:1, 15:1 to 35:1, 15:1 to 40 :1, 20:1 to 25:1, 20:1 to 30:1, 20:1 to 35:1, 20:1 to 40:1, 25:1 to 30:1, 25:1 to 35:1, 25:1 to 40:1, 30:1 to 35:1, 30:1 to 40:1, 35:1 to 40:1.

In some embodiments, gemcitabine or the pharmaceutically acceptable salt thereof is present in the aqueous interior, and the salt solution in the aqueous interior is a sulfate salt solution, or a combination of a sulfate salt solution and a buffer salt solution (e.g., phosphate buffer solution), or a combination of a chloride salt solution and a buffer salt solution (e.g., phosphate buffer solution).

In some embodiments, the molar concentration of the salt solution in the aqueous interior is 100 to 800 mM, preferably 200 to 600 mM.

In some embodiments, the salt solution in the aqueous interior is an ammonium sulfate solution; preferably, the concentration of the ammonium sulfate solution is 100 to 800 mM, preferably 200 to 600 mM, more preferably 200 to 400 mM, more preferably 300 to 400 mM.

In some embodiments, the salt solution in the aqueous interior is a mixed solution of sodium sulfate and a buffer slat of phosphate, or a mixed solution of sodium chloride and a salt of phosphate buffer, or a mixed solution of ammonium sulfate and a salt of phosphate buffer; preferably, the concentration of sodium sulfate, sodium chloride or ammonium sulfate in the mixed solution is 100 to 800 mM (preferably 200 to 600 mM, more preferably 200 to 500 mM, more preferably 250 to 500 mM), and the concentration of the salt of phosphate buffer is 100 to 200 mM.

In some embodiments, the liposome is single layered, and has an average particle size of 10 to 200 nm, preferably 30 to 150 nm, more preferably 50 to 100 nm.

In some embodiments, the concentration of gemcitabine or the pharmaceutically acceptable salt thereof in the liposome is 0.1 to 3 mg/ml, more preferably 0.1 to 2.5 mg/ml, 0.1 to 2 mg/ml, for example, 0.1 to 0.5 mg/ml, 0.1 to 1.0 mg/ml, 0.1 to 1.5 mg/ml, 0.1 to 2.0 mg/ml, 0.1 to 2.5 mg/ml, 0.1 to 3.0 mg/ml, 0.5 to 1.0 mg/ml, 0.5 to 1.5mg/ ml, 0.5 to 2.0 mg/ml, 0.5 to 2.5 mg/ml, 0.5 to 3.0 mg/ml, 1.0 to 1.5 mg/ml, 1.0 to 2.0 mg/ml, 1.0 to 2.5 mg/ml, 1.0 to 3.0 mg/ml, 1.5 to 2.0 mg/ml, 1.5 to 2.5 mg/ml, 1.5 to 3.0 mg/ml, 2.0 to 2.5 mg/ml, 2.0 to 3.0 mg/ml, 2.5 to 3.0 mg/ml.

In some embodiments, the phospholipid is one or two selected from natural phospholipids and derivatives thereof, including lecithin, cephalin, soybean lecithin, phosphatidylethanolamine, phosphatidylcholine, phosphatidylglycerol, phosphatidylserine, sphingomyelin, cardiolipin, etc. (including hydrogenated phospholipids obtained by hydrogenating the above phospholipids). when the liposome contains one type of phospholipids, it is preferably the one selected from the group consisting of a neutral phospholipid, a negatively charged phospholipid and sphingomyelin, preferably phosphatidylcholine or sphingomyelin, such as hydrogenated soybean phosphatidylcholine (HSPC), distearoylphosphatidylcholine (DSPC), dipalmitoylphosphatidylcholine (DPPC), sphingomyelin (SM), hydrogenated sphingomyelin (HSM);

when there contained two types of phospholipids, it is preferably a combination of a neutral phospholipid and a negatively charged phospholipid, more preferably phosphatidylcholine and a phosphatidylglycerol, such as DSPC and distearoylphosphatidylglycerol (DSPG), HSPC and DSPG, or HSPC and dipalmitoylphosphatidylglycerol (DPPG). When the phospholipid comprises one or more of sphingomyelin, a hydrogenated sphingomyelin and a negatively charged phospholipid, preferably, the liposome membrane does not comprise the long-circulating functional material, that is, the mass ratio of the long-circulating functional material is 0%.

In some preferred embodiments, the phospholipid is one or more of hydrogenated soybean phosphatidylcholine (HSPC), distearoylphosphatidylcholine (DSPC), dipalmitoylphosphatidylcholine (DPPC), distearoylphosphatidylglycerol (DSPG), dipalmitoylphosphatidylglycerol (DPPG), sphingomyelin (SM) and hydrogenated sphingomyelin (HSM); the phospholipid is preferably hydrogenated soybean phosphatidylcholine (HSPC).

When distearoylphosphatidylglycerol (DSPG), dipalmitoylphosphatidylglycerol (DPPG), sphingomyelin (SM) or hydrogenated sphingomyelin (HSM) is used as the phospholipid, the liposome membrane does not comprise the long-circulating functional material.

In some preferred embodiments, the phospholipid is one or two selected from the group consisting of hydrogenated soybean phosphatidylcholine (HSPC), dipalmitoylphosphatidylglycerol (DPPG), sphingomyelin (SM) and hydrogenated sphingomyelin (HSM); when dipalmitoylphosphatidylglycerol (DPPG), sphingomyelin (SM) or hydrogenated sphingomyelin (HSM) is used as the phospholipid, the liposome membrane does not comprise the long-circulating functional material.

In some preferred embodiments, the phospholipid is selected from the group consisting of hydrogenated soybean phosphatidylcholine, sphingomyelin, and a combination of hydrogenated soybean phosphatidylcholine and dipalmitoylphosphatidylglycerol.

In some embodiments, the long-circulating functional material is one or more selected from the group consisting of polyethylene glycol (PEG), polyethylene glycol-vitamin E succinate (TPGS), polyethylene glycol-cholesterol (PEG-Chol), polyethylene glycol-modified distearoylphosphatidylethanolamine (MPEG-DSPE), polyethylene glycol-modified dimyristoylphosphatidylethanolamine (MPEG-DMPE) and polyethylene glycol-modified dipalmitoylphosphatidylethanolamine (MPEG-DPPE), preferably a polyethylene glycol-modified phospholipid which is the same type as the phospholipid that constitutes the liposome membrane. The molecular weight of the polyethylene glycol is 500 to 10,000 Daltons, preferably 2000 to 5000 Daltons.

In some preferred embodiments, the long-circulating functional material is polyethylene glycol-modified distearoylphosphatidylethanolamine (MPEG-DSPE), such as MPEG2000-DSPE.

The advantages of liposome compositions in reducing toxicity and enhancing efficacy are closely related to their release in the blood. Further research on liposome compositions found that the type and amount of salt solution affect the drug release behavior of liposomes in the blood. In some embodiments, the salt may be one or more selected from salts containing $Na^+$, $K^+$, $NH_4^+$ or $H^+$, and the acid ions constituting the salt comprise one or more organic acid radicals or inorganic acid radicals, including but not limited to sulfate, carbonate, chloride, hydrogen phosphate, dihydrogen phosphate, sucrose octasulfate, citrate, gluconate, methanesulfonate, acetate, oxalate, 4-hydroxyethyl piperazine ethanesulfonate, preferably one or more selected from the group consisting of chloride, sulfate, sucrose octasulfate, acetate, hydrogen phosphate and dihydrogen phosphate, more preferably sulfate, or a combination of sulfate, chloride, phosphate, hydrogen phosphate and dihydrogen phosphate.

In some embodiments, the salt is preferably ammonium sulfate, or a combination of sodium sulfate and a salt of phosphate buffer, or a combination of sodium chloride and a salt of phosphate buffer, or a combination of ammonium sulfate and a salt of phosphate buffer.

In some embodiments, the present invention provides a liposome of gemcitabine or a pharmaceutically acceptable salt thereof, which comprises a liposome membrane and an aqueous interior encapsulated by the liposome membrane, wherein,

the components constituting the liposome membrane comprise hydrogenated soybean phosphatidylcholine (HSPC), cholesterol and polyethylene glycol-modified distearoylphosphatidylethanolamine (MPEG2000-DSPE); the aqueous interior comprises gemcitabine hydrochloride, a salt solution, the salt solution is an ammonium sulfate solution, or a mixed solution of sodium sulfate and a salt of phosphate buffer, or a mixed solution of sodium chloride and a salt of phosphate buffer, or a mixed solution of ammonium sulfate and a salt of phosphate buffer; and,

the weight ratio of HSPC to cholesterol is 2:1 to 20:1, preferably 2:1 to 10:1, more preferably 2:1 to 8:1, such as 2:1 to 3:1 or 7:1 to 8:1;

MPEG2000-DSPE accounts for 0% to 30%, preferably 0% to 20%, preferably 10% to 20%, preferably 0% to 15%, preferably 0.01% to 15%, by mass of total lipids;

the weight ratio of total lipids to gemcitabine hydrochloride in the liposome is 10:1 to 50:1;

the molar concentration of the salt solution in the aqueous interior is 50 to 800 mM.

In some embodiments, the salt solution in the aqueous interior is an ammonium sulfate solution; the concentration of the ammonium sulfate solution is 300 to 400 mM.

In some embodiments, the liposome of gemcitabine or the pharmaceutically acceptable salt thereof provided by the present invention can be an injection, and aqueous exterior of the injection optionally comprises an osmotic pressure regulator, the osmotic pressure regulator is one or more selected from the group consisting of glucose, sucrose, sorbitol, mannitol, sodium chloride, phosphate, sulfate, acetate, glycerol, histidine and hydrochloride thereof, glycine and hydrochloride thereof, lysine, serine, glutamic acid, arginine or valine.

In some embodiments, the liposome has an encapsulation efficiency of above 85%, such as above 95%, above 96%, above 97%, above 98%, above 99%, above 99.5%.

In a second aspect, the present application further provides a method for preparing the gemcitabine liposome, which comprises the following steps:

51, preparation of an aqueous phase: dissolving gemcitabine or a pharmaceutically acceptable salt thereof in a salt solution with a concentration of 50 to 800 mM to obtain the aqueous phase;

S2, preparation of a lipid phase: dissolving phospholipid and cholesterol in a weight ratio of 2:1 to 20:1 in a solvent to obtain the lipid phase, wherein the solvent is one or more selected from the group consisting of ethanol, methanol, ethyl acetate, chloroform, dichloromethane and diethyl ether;

S3, preparation of a drug-loaded liposome: mixing the aqueous phase obtained in S1 and the lipid phase obtained in S2 by organic solvent injection method, thin-film hydration method, reverse-phase evaporation method, emulsification method, freeze-thaw cycles, pipeline mixing method, or microfluidic method to obtain a primary emulsion of liposome containing gemcitabine or the pharmaceutically acceptable salt thereof and a salt solution of an aqueous interior;

S4, granulation: granulating the primary emulsion of the drug-loaded liposome by using one or more selected from the group consisting of shearer, homogenizer and extruder to reach a target particle size;

S5, removal of free drugs: removing free drugs by one or more treatments selected from filtration, centrifugation, dialysis, ultrafiltration, concentration, and etc. to obtain the drug-loaded liposome comprising gemcitabine or the pharmaceutically acceptable salt thereof;

optionally, adding a long-circulating functional material when preparing the lipid phase in step S2.

In some embodiments, the salt solution in step S1 is preferably an ammonium sulfate solution. In some embodiments, the molar concentration of the ammonium sulfate solution is 50 to 800 mM, preferably 50 to 600 mM, preferably 50 to 400 mM, preferably 50 to 200 mM, preferably 50 to 100 mM. In some embodiments, the molar concentration of the ammonium sulfate solution is 100 to 800 mM, preferably 200 to 600 mM, more preferably 200 to 400 mM, more preferably 300 to 400 mM.

In some embodiments, in step S1, a pH-adjusting agent can be added to adjust the pH of the aqueous phase. In some embodiments, the pH adjusting agent is selected from the group consisting of hydrochloric acid, acetic acid, phosphoric acid, sodium hydroxide, potassium hydroxide, calcium hydroxide, and the like.

In some embodiments, the phospholipid in step S2 is HSPC.

In some embodiments, the long-circulating functional material in step S2 is MPEG2000-DSPE.

In some embodiments, in step S2, the weight ratio of the phospholipid to cholesterol is 2:1 to 15:1, preferably 2:1 to 10:1, preferably 2.5:1 to 10:1, more preferably 5 :1 to 8:1.

In some embodiments, in step S2, the long-circulating functional material added accounts for 0% to 30%, preferably 0% to 20%, preferably 10% to 20%, preferably 0% to 15%, preferably 0.01% to 15%, by mass of total lipids.

In some embodiments, in step S3, at a lipid-to-water ratio of 1:1 to 1:20, the aqueous phase prepared in S1 and the lipid phase prepared in S2 are mixed to obtain the drug-loaded liposome; preferably, the lipid-to-water ratio is 1:3, 1:5, 1:7, 1:10 or 1:20.

In some embodiments, step S3 is conducted at or above the phase transition temperature of the lipid.

In some embodiments, step S3 is conducted at 30 to 70°C (e.g., 30 to 40°C, 50 to 60°C, 60 to 70°C, or 60 to 65°C).

In some embodiments, in step S4, the primary emulsion of the drug-loaded liposome is granulated to reach an average particle size of 10 to 200 nm, such as 30 to 200 nm, 50 to 200 nm, 150 to 200 nm, 10 to 150 nm, 30 to 150 nm, 50 to 150 nm, 100 to 150 nm, 10 to 100 nm, 30 to 100 nm, 50 to 100 nm, 10 to 50 nm, 30 to 50 nm or 10 to 30 nm, preferably 50 to 100 nm.

In some embodiments, the drug-loaded liposome obtained in step S4 is diluted with a sucrose-histidine solution and ultrafiltered to remove free gemcitabine; preferably, the sucrose-histidine solution is 10% sucrose-0.155% histidine. (w/v, pH6.5).

The concentration of gemcitabine or the pharmaceutically acceptable salt thereof in the gemcitabine liposome prepared by the method of the present invention is 0.1 to 3 mg/ml, preferably 0.1 to 2 mg/ml, such as 0.1 to 1.5 mg/ml, 0.1 to 1 mg/ml or 0.1 to 0.5 mg/ml.

The encapsulation efficiency of the gemcitabine liposome prepared by the method of the present invention is more than 85%, more preferably more than 95%, such as more than 96%, more than 97%, more than 98%, more than 99%.

In the gemcitabine liposome prepared by the method of the present invention, the weight ratio of total lipids to gemcitabine or the pharmaceutically acceptable salt thereof is 1:1 to 100:1, preferably 5:1 to 80:1; more preferably 10:1 to 50:1, more preferably 15:1 to 40:1.

In a third aspect, the present application provides another method for preparing the gemcitabine liposome, which comprises the following steps:

SI, preparation of an aqueous phase: preparing a salt solution having a concentration of 50 to 800 mM as the aqueous phase;

SII, preparation of a lipid phase: dissolving a phospholipid and cholesterol in a weight ratio of 2:1 to 20:1 in a solvent to obtain the lipid phase, wherein the solvent is one or more selected from the group consisting of water, ethanol, methanol, ethyl acetate, chloroform, dichloromethane and diethyl ether;

SIII, preparation of a primary emulsion of blank liposome: mixing the aqueous phase obtained in SI and the lipid phase obtained in SII in appropriate amounts by organic solvent injection method, thin-film hydration method, reverse-phase evaporation method, emulsification method, freeze-thaw cycles, pipeline mixing method, or microfluidic method to obtain the primary emulsion of blank liposome;

SIV, granulation: granulating the primary emulsion of blank liposome by one or more selected from the group consisting of shear, homogenizer and extruder to reach a target particle size;

SV, removal of organic solvent: removing the organic solvent by one or more treatments selected from the group consisting of filtration, centrifugation, dialysis, ultrafiltration, concentration, and etc. to obtain a blank liposome;

SVI, preparation of an API solution: dissolving gemcitabine or a pharmaceutically acceptable salt thereof in water or the aqueous phase of step SI which is diluted or undiluted to prepare the API solution;

SVII, preparation of a drug-loaded liposome: heating and incubating the obtained blank liposome and the API solution at 30°C to 75°C for 5 to 60 minutes to obtain the drug-loaded liposome;

SVIII, removal of free drugs: removing the free drugs by one or more treatments selected from filtration, centrifugation, dialysis, ultrafiltration, concentration, and etc. to obtain the drug-loaded liposome containing gemcitabine or the pharmaceutically acceptable salt thereof;

optionally, adding a long-circulating functional material when preparing the API solution in step SVI or preparing the drug-loaded liposome in step SVII.

In some embodiments, the long-circulating functional material optionally added when preparing the API solution in step SVI or preparing the drug-loaded liposome in step SVII is MPEG2000-DSPE.

In some embodiments, the salt solution in step SI is an ammonium sulfate solution, a mixed solution of sodium sulfate and a salt of phosphate buffer, a mixed solution of sodium chloride and a salt of phosphate buffer, or a mixed solution of ammonium sulfate and a salt of phosphate buffer. In some embodiments, the concentration of the ammonium sulfate solution is 100 to 800 mM, preferably 200 to 600 mM, more preferably 200 to 400 mM, more preferably 300 to 400 mM. In some embodiments, the concentration of sodium sulfate, sodium chloride or ammonium sulfate in the mixed solution is 100 to 800 mM (preferably 200 to 600 mM, more preferably 200 to 500 mM, more preferably 250 to 500 mM), and the concentration of the salt of phosphate buffer is 100 to 200 mM.

In some embodiments, the phospholipid in step SII is selected from the group consisting of sphingomyelin, hydrogenated soybean phosphatidylcholine, and a combination of hydrogenated soybean phosphatidylcholine and dipalmitoylphosphatidylglycerol. In some embodiments, the weight ratio of the phospholipid to cholesterol in step SII is 2:1 to 15:1, preferably 2:1 to 10:1, more preferably 2:1 to 8:1, such as 2:1 to 3:1 or 7:1 to 8:1.

In some embodiments, the solvent in step SII is ethanol or a mixed solution of ethanol and water.

In some embodiments, in step SIII, the lipid phase prepared in step SII is added at an oil-to-water ratio of 1:1 to 1:20 (e.g., 1:1 to 1:10, particularly, such as 1:3, 1:5, 1:10, or 1:20) to an appropriate amount of the aqueous phase prepared in step SI, and stirred at or above the lipid phase transition temperature to obtain the primary emulsion of blank liposome.

In some embodiments, step SIII comprises the following steps: stirring and emulsifying at 30 to 75°C (e.g., 30 to 40°C, 50 to 60°C, or 60 to 70°C) to obtain the primary emulsion of blank liposome.

In some embodiments, the primary emulsion of blank liposome prepared in step SIV has an average particle size of 10 to 200 nm, such as 30 to 200 nm, 50 to 150 nm, 50 to 100 nm, 60 to 80 nm, specifically such as 60 nm, 65 nm, 70 nm, 75 nm, or 80 nm.

In some embodiments, the concentration of the API solution in step SVI is 0.01 to 0.5 g/ml, such as 0.01 to 0.2 g/ml, 0.05 to 0.2 g/ml.

In some embodiments, in step SVI, a pH adjusting agent can be added to adjust the pH of the API solution. In some embodiments, the pH adjusting agent is selected from the group consisting of hydrochloric acid, acetic acid, phosphoric acid, sodium hydroxide, potassium hydroxide, calcium hydroxide, and the like.

In some embodiments, in step SVII, the weight ratio of gemcitabine or the pharmaceutically acceptable salt thereof to total lipids in the blank liposome is 2:1 to 1:10, preferably 2:1 to 1:5, for example 1:1 to 1:5, for another example 1:1 to 1:3, for another example 1:1 to 1:2; particularly, such as 1:1, 1:1.1, 1:1.2, 1:1.3, 1:1.4, 1 :1.5, 1:1.6, 1:1.7, 1:1.8, 1:1.9, 1:2.0.

In some embodiments, the incubation temperature in step SVIIis 50 to 75°C (e.g., 65 to 70°C).

In some embodiments, the incubation time in step SVII is 10 min to 45 min.

In some embodiments, the long-circulating functional material added in step SVI or SVII accounts for 0% to 15%, such as 0%, or 0.01% to 15%, particularly, such as 0.01%, 0.05%, 1%, 2%, 3%, 4%, 5%, 6%, 7%, 8%, 9%, 10%, 11%, 12%, 13%, 14% or 15%, by mass of total lipids added in step SII and SVI or SVII.

In some embodiments, in step SVIII, the drug-loaded liposome obtained in step SVII is added to the aqueous phase described in SI and/or a sucrose-histidine solution, and ultrafiltrated to remove free gemcitabine; preferably, the sucrose-histidine solution is 10% sucrose-0.155% histidine (w/v, pH 6.5).

The concentration of gemcitabine or the pharmaceutically acceptable salt thereof in the gemcitabine liposome prepared by the method of the present invention is 0.1 to 3 mg/ml, preferably 0.5 to 3 mg/ml, such as 0.5 to 2.5 mg/ml.

The encapsulation efficiency of the gemcitabine liposome prepared by the method of the present invention is more than 85%, more preferably more than 95%, such as more than 96%, more than 97%, more than 98%, more than 99%.

In the gemcitabine liposome prepared by the method of the present invention, the weight ratio of total lipids to gemcitabine or the pharmaceutically acceptable salt thereof is 1:1 to 100:1, preferably 5:1 to 80:1; more preferably 10:1 to 50:1, more preferably 15:1 to 40:1.

In the fourth aspect, the present invention provides a liposome preparation, which comprises the gemcitabine liposome described in any one of items of the first aspect, and one or more pharmaceutically acceptable carriers.

In some embodiments, the liposome formulation comprises an osmotic pressure regulator, such as a sucrose-histidine solution, preferably a 10% sucrose-histidine solution, preferably a 10% sucrose-0.155% histidine solution.

In some embodiments, the liposome formulation is an injection.

In another aspect, the present application provides use of the gemcitabine liposome described in any one of items of the first aspect or the liposome preparation described in any one of items of the fourth aspect in the manufacture of a medicament for treating a tumor.

In another aspect, the present invention provides use of the gemcitabine liposome according to any one of items of the first aspect or the liposome preparation according to any one of items of the fourth aspect in the manufacture of an anti-tumor drug.

In some embodiments, the tumor is selected from the group consisting of pancreatic cancer, breast cancer, non-small cell lung cancer and ovarian cancer.

In another aspect, the present application provides a method for treating a tumor, which comprises a step of administering to a subject in need thereof with an effective amount of the gemcitabine liposome described in any one of items of the first aspect or the liposome preparation according to any one of items of the fourth aspect.

In some embodiments, the tumor is selected from the group consisting of pancreatic cancer, breast cancer, non-small cell lung cancer and ovarian cancer.

Definition

Unless otherwise defined, all technical and scientific terms used herein have the same meaning as commonly understood by those of ordinary skilled in the art. The terminology used herein in the description of the present invention is for the purpose of describing specific embodiments only and is not intended to limit the present invention.

As used herein, the term "phosphatidylethanolamine pegol" refers to polyethylene glycol-modified phosphatidylethanolamine, such as polyethylene glycol-modified distearoylphosphatidylethanolamine (MPEG-DSPE), polyethylene glycol-modified dimyristoylphosphatidylethanolamine (MPEG-DMPE), polyethylene glycol-modified dipalmitoylphosphatidylethanolamine (MPEG-DPPE), etc. In the present invention, MPEG-DSPE, such as MPEG2000-DSPE, is particularly preferred.

As used herein, the term "lipid phase" may also be referred to as "oil phase" or "organic phase".

As used herein, the term "API" refers to "gemcitabine or pharmaceutically acceptable salts thereof," and in some embodiments, specifically gemcitabine hydrochloride.

As used herein, the term "total lipids" comprise phospholipids, cholesterol, and long-circulating functional materials contained in the liposome.

As used herein, the term "pharmaceutically acceptable carrier" refers to a carrier that is pharmacologically and/or physiologically compatible with the subject and the active ingredient and is well known in the art (see, for example, Remington's Pharmaceutical Sciences. Edited by Gennaro AR, 19th ed. Pennsylvania: Mack Publishing Company, 1995).

As used herein, the term "treatment" refers to a method performed to obtain a beneficial or desired clinical result. For the purposes of the present invention, the beneficial or desired clinical result includes, but is not limited to, alleviation of symptom, reduction of disease extent, stabilization (i.e., no worsening) of disease state, delaying or slowing disease progression, ameliorating or alleviating disease status, and relief of symptoms (whether partial or complete), whether detectable or undetectable. In addition, "treatment" may also refer to prolonging survival compared with expected survival if no treatment is received.

As used herein, the term "subject" refers to a mammal, such as a primate mammal, such as human. In certain embodiments, the subject (e.g., human) has a tumor, or is at risk of suffering from a disease described above.

As used herein, the term "effective amount" refers to an amount sufficient to obtain, at least partially, the desired effect. For example, a prophylactically effective amount refers to an amount sufficient to prevent, arrest, or delay the occurrence of a disease (e.g., a tumor); a therapeutically effective amount refers to an amount sufficient to cure or at least partially prevent an existing disease and complications thereof in a patient who is suffering from the disease. Determining such effective amounts is well within the capabilities of those skilled in the art. For example, the amount effective for therapeutic use will depend on the severity of the disease to be treated, the overall status of the patient's own immune system, the patient's general condition such as age, weight and gender, the manner in which the drug is administered, and other treatments administered concurrently, etc.

The terms "cancer" and "tumor" are used interchangeably and refer to a large group of diseases characterized by the uncontrolled growth of abnormal cells in the body. Unregulated cell division may lead to the formation of malignant tumors or cells that invade adjacent tissues and may metastasize to distant parts of the body via the lymphatic system or bloodstream. Cancers include benign and malignant cancers as well as dormant tumors or micrometastases. Cancers also include blood tumors, especially hematological malignancies.

Beneficial effects of the present invention

The present invention provides a method for preparing a liposome of gemcitabine or a pharmaceutically acceptable salt thereof, wherein the liposome is prepared by optimizing and controlling the ratio of phospholipid to cholesterol, the proportion of long-circulating functional material in total lipids, the ratio of total lipids to the drug, and the type and concentration of the salt solution in aqueous interior, and has high encapsulation efficiency, good storage stability, strong filtration, not easy to leak, stable and controllable release, significantly prolonged half-life, improved therapeutic effect and reduced toxic and side effects. After intravenous injection of the liposome, it can avoid the rapid metabolic degradation of free gemcitabine, significantly prolong the circulation time of gemcitabine in the body, enhance the tumor tissue/cell targeting, and at the same time reduce the dose of gemcitabine, improve the efficacy and reduce toxic and side effects, thereby meeting the clinical needs of improving efficacy and reducing toxicity. In addition, the liposome provided by the present invention has simple prescription and preparation process, which is feasible to scale-up production and clinical promotion and application.

## Brief Description of the Drawings

The drawings described herein are used to provide further understanding of the present invention and constitute a part of the present application. The illustrative examples of the present invention and their descriptions are used to explain the present invention and do not constitute an improper limitation on the present invention. In the attached drawings:

Fig. 1 shows the drug-time curves of the liposome group and the conventional injection group in Balb/c female tumor-bearing mice.

Fig. 2 shows the diagram of tumor volume changes of the liposome group and the conventional injection group in 4T1Balb/c mice pharmacodynamic study.

Fig. 3 shows the diagram of BxPC-3 efficacy results.

## Specific Models for Carrying Out the present Invention

The technical solutions in the embodiments of the present invention will be clearly and completely described below with reference to the accompanying drawings in the examples of the present invention. Obviously, the examples as described are only some of the examples of the present invention, rather than all examples. The following description of at least one exemplary example is merely illustrative in nature and is in no way intended to limit the present invention and application or use thereof. Based on the examples of the present invention, all other examples obtained by those of ordinary skill in the art without making creative efforts fall within the scope of protection of the present invention.

Example 1: Preparation of gemcitabine liposome

The preparation process was as follows:

1. Preparation of lipid phase

16.6g of hydrogenated soybean phosphatidylcholine, 2.0g of cholesterol, and 4.3g of MPEG2000-DSPE were weighed and dissolved in 20 ml of anhydrous ethanol by heating to 60°C, and kept at the temperature for later use.

2. Preparation of aqueous phase

1.56g of disodium hydrogen phosphate and 0.15g of sodium dihydrogen phosphate monohydrate were weighed and dissolved in 3000g of water to obtain 4mM phosphate buffer with a pH of 7.78, which was heated to 60°C and kept at the temperature for later use.

3. Preparation of blank liposome

At 60°C, the lipid phase was injected into the aqueous phase at a speed of 60 ml/min. After the injection was completed, stirring was continued for 30 minutes to obtain a primary emulsion of liposome, which extruded through a homogeneous extrusion machine so that the primary emulsion of liposome had an average particle diameter of 68nm, and then the ethanol was removed by ultrafiltration system to obtain 134g of blank liposome that did not contain drug.

4. Preparation of drug-loaded liposome

4.1 Preparation of PBS buffer

20.41g of sodium chloride, 2.88g of disodium hydrogen phosphate and 0.51g of sodium dihydrogen phosphate monohydrate were weighed and dissolved in 234g of water to obtain a PBS buffer.

4.2 Preparation of API solution

3.25g of gemcitabine hydrochloride and 0.22g of sodium hydroxide were weighed and dissolved in 19.0g of water for injection, mixed with 13.50g of the PBS buffer prepared in (4.1), then heated to 70°C and kept at the temperature for later use.

4.3 Drug loading

35.4g of the blank liposome prepared in (3) and 0.22g of sodium hydroxide were weighed, heated to 70°C, and then heated with the API solution prepared in (4.2) for 10 minutes to load the drug, immediately cooled to 40°C, and diluted by adding 1016mM sucrose/37mM histidine solution, and then subjected to ultrafiltration dialysis by using 275mM sucrose/10 mM histidine, and the aqueous exterior was replaced with the ultrafiltration dialysate to obtain the title liposome. The liposome obtained had an average particle size of 77.7 nm, the concentration of gemcitabine hydrochloride was 1.12 mg/ml, the encapsulation efficiency was 95.23%, the mass ratio of total lipids to API was 34:1, and MPEG2000-DSPE accounts for 18.9% by mass of total lipids.

Example 2: Preparation of gemcitabine liposome

(1) Preparation of lipid phase

2.75g of hydrogenated soybean phosphatidylcholine, 0.91g of cholesterol, and 0.86g of MPEG2000-DSPE were weighed and dissolved in 20 ml of anhydrous ethanol by heating to 60°C, and kept at the temperature for later use.

(2) Preparation of aqueous phase

1.86g of gemcitabine hydrochloride and 0.25g of sodium hydroxide were weighed and dissolved in 188.73g of an ammonium sulfate solution (73mM, obtained by dissolving 1.82g of ammonium sulfate in 188.73g of water), and kept at the temperature at 60°C for later use.

(3) Preparation of drug-loaded liposome

Under the same temperature conditions, with a total flow rate of 12ml/min and an oil-to-water ratio of 1:7, the lipid phase and the aqueous phase were mixed separately through microfluidic pipelines to obtain a drug-loaded liposome, which was diluted 10 times by adding 10% sucrose-0.155% histidine (w/v, adjusted to pH 6.5 by adding dilute hydrochloric acid) solution, then subjected to ultrafiltration dialysis and concentration using a tangential flow ultrafiltration device to remove free drug and organic solvent (the dialysate was a pH6.5 10% sucrose-0.155% histidine solution). After dialysis, gemcitabine liposomes were obtained. The liposomes obtained had an average particle size of 152.20 nm, the concentration of gemcitabine hydrochloride was 0.38 mg/ml, the encapsulation efficiency was 97.79%, the mass ratio of total lipids to API was 38:1, and MPEG2000-DSPE accounts for 19% by mass of total lipids.

Example 3: Preparation of gemcitabine liposome

(1) Preparation of lipid phase

12.29g of sphingomyelin and 4.49g of cholesterol were weighed and dissolved in 81.72g of anhydrous ethanol by heating to 60°C, and kept at the temperature for later use.

(2) Preparation of water phase

225.88g of ammonium sulfate was weighed and dissolved in 5000g of water to prepare a 342 mM ammonium sulfate buffer salt solution, which was kept at 60°C for later use.

(3) Preparation of blank liposome

Under the same temperature conditions, the lipid phase prepared in (1) was injected into 800ml of the water phase prepared in (2) at a speed of 100ml/min, continuously heated and stirred for 45 minutes to obtain a primary emulsion of liposome, and the primary emulsion of liposome obtained was subjected to extrusion to reach an average particle size of about 75 nm, the organic solvent was removed by dialysis using a tangential flow ultrafiltration device to obtain a blank liposome with an aqueous interior containing 342 mM ammonium sulfate buffer salt solution.

(4) Preparation of drug-loaded liposome

4.11g of gemcitabine hydrochloride and 5.50g of sodium hydroxide were weighed and dissolved in 52.28g of water by heating to 65°C, then added into 100g of the blank liposome prepared in (3) incubated at 65°C, and heated for loading drug for 30 minutes to obtain a drug-loaded liposome, which was diluted 5 times by adding 10% sucrose-0.155% histidine (w/v, pH 6.5), and then dialyzed using a tangential flow ultrafiltration device to remove free gemcitabine and obtain a gemcitabine liposome. The liposome obtained had an average particle size of 74.37 nm, the concentration of gemcitabine hydrochloride was 1.39 mg/ml, the encapsulation efficiency was 98.89%, and the mass ratio of total lipids to API was 40:1.

Example 4: Preparation of gemcitabine liposome

(1) Preparation of lipid phase

8.29g of hydrogenated soybean phosphatidylcholine and 1.10g of cholesterol were weighed and dissolved in 60 ml of anhydrous ethanol by heating to 60°C, and kept at the temperature for later use.

(2) Preparation of water phase

112.00g of sodium sulfate, 23.01g of disodium hydrogen phosphate and 5.24g of sodium dihydrogen phosphate (monohydrate) were weighed and dissolved in 2000.5g of water by heating to 60°C to obtain a 394 mM sodium sulfate-100 mM phosphate buffer, which was kept at 60°C for later use.

(3) Preparation of blank liposome

Under the same temperature conditions, the lipid phase prepared in (1) was injected into 300 ml of the water phase prepared in (2) at an injection speed of 60 ml/min, continuously heated and stirred for 10 minutes to obtain a primary emulsion of liposome, and the primary emulsion of liposome obtained was extruded to reach an average particle size of 70 nm, and then dialyzed with a tangential flow ultrafiltration device to remove the organic solvent. After concentration, 64g of blank liposomes with an aqueous interior containing the 394 mM sodium sulfate-100 mM phosphate buffer solution was obtained.

(4) Preparation of drug-loaded liposome

2.88g of gemcitabine hydrochloride, 0.49g of sodium hydroxide and 0.44g of MPEG2000-DSPE were weighed, heated to 60°C and dissolved in 27.00g of triple dilution of the aqueous phase prepared in (2), then added into 30g of the blank liposome prepared in (3) incubated at 70°C, and heated for loading drug at 70°C for 10 minutes to obtain a drug-loaded liposome, which was 5-fold diluted by adding the aqueous phase prepared in (2) and dialyzed by a tangential flow ultrafiltration device to remove free gemcitabine and obtain a gemcitabine liposome. The liposome obtained had an average particle size of 82.30 nm, the gemcitabine hydrochloride concentration was 2.06 mg/ml, the encapsulation efficiency was 99.12%, the mass ratio of total lipids to API was 15:1, and MPEG2000-DSPE accounts for 12.5% by mass of total lipids.

Example 5: Preparation of gemcitabine liposome

(1) Preparation of lipid phase

8.23g of hydrogenated soybean phosphatidylcholine and 1.03g of cholesterol were weighed and dissolved in 60 ml of anhydrous ethanol by heating to 60°C, and kept at the temperature for later use.

(2) Preparation of water phase

74.00g of ammonium sulfate, 22.99g of disodium hydrogen phosphate, and 5.25g of sodium dihydrogen phosphate (monohydrate) were weighed and dissolved in 2000g of purified water to obtain a 280 mM ammonium sulfate-100mM phosphate buffer solution, which was kept at 60°C for later use.

(3) Preparation of blank liposome

Under the same temperature conditions, the lipid phase prepared in (1) was injected into the aqueous phase prepared in (2) at an injection speed of 60 ml/min, continuously heated and stirred for 10 minutes to obtain a primary emulsion of liposome, and the obtained primary emulsion of liposome was subjected to extrusion to reach an average particle size of about 75 nm, the organic solvent was removed by dialysis using a tangential flow ultrafiltration device, and concentration was performed to obtain 92g of a blank liposome with the 280 mM ammonium sulfate-100 mM phosphate buffer solution as aqueous interior.

(4) Preparation of drug-loaded liposome

1.16g of gemcitabine hydrochloride, 0.16g of sodium hydroxide and 38.34 mg of MPEG2000-DSPE were weighed, heated to 70°C and dissolved in 18.18g of the aqueous phase prepared in (2), then added into 40g of the blank liposome prepared in (3) incubated at 70°C, and heated for loading drug for 10 minutes to obtain a drug-loaded liposome, which was dialyzed by a tangential flow ultrafiltration device to remove free gemcitabine and obtain a gemcitabine liposome. The obtained liposome had an average particle size of 79.38 nm, the concentration of gemcitabine hydrochloride was 0.94 mg/ml, the encapsulation efficiency was 98.35%, the ratio of total lipids to API was 33:1, and MPEG2000-DSPE accounts for 1.7% by mass of total lipids.

Example 6: Preparation of gemcitabine liposome

(1) Preparation of lipid phase

28.43g of hydrogenated soybean phosphatidylcholine and 3.61g of cholesterol were weighed and dissolved in 200ml of anhydrous ethanol by heating to 60°C, and kept at the temperature for later use.

(2) Preparation of water phase

108.00g of NaCl, 46.00g of disodium hydrogen phosphate and 10.49g of sodium dihydrogen phosphate (monohydrate) were weighed and dissolved in 4000g of water to prepare a 462 mM NaCl-100 mM phosphate buffer solution, which was kept at 60°C for later use.

(3) Preparation of blank liposome

The lipid phase prepared in (1) was injected into 1045.5g of the water phase prepared in (2), heated and stirred for 10 minutes to obtain a primary emulsion of liposome. After the primary emulsion of liposome was granulated to about 75nm through an extruder, the ethanol was removed by ultrafiltration to obtain a blank liposome with the 462 mM NaCl-100 mM phosphate buffer solution as aqueous interior.

(4) Preparation of drug-loaded liposome

23.05g gemcitabine hydrochloride, 3.43g MPEG2000-DSPE, and 3.08g sodium hydroxide were weighed, heated to 70°C and dissolved in 328.00g of the 462 mM NaCl-100 mM phosphate buffer solution prepared in (2), then added into 310g of the blank liposome prepared in (3) incubated at 65°C, then heated for loading drug for 10 minutes to obtain a drug-loaded liposome. After the drug-loaded liposome was subjected to ultrafiltration to remove free drug, the obtained liposome had an average particle size of 86.31 nm, the concentration of gemcitabine hydrochloride was 2.34 mg/ml, the encapsulation efficiency was 95.14%, the mass ratio of total lipids to API was 27:1, and MPEG2000-DSPE accounts for 12% by mass of total lipids.

Example 7: Preparation of gemcitabine liposome

The preparation methods for (1) lipid phase, (2) aqueous phase and (3) blank liposome were the same as those in Example 5.

(4) Preparation of drug-loaded liposome

1.16g of gemcitabine hydrochloride, 0.16g of sodium hydroxide and 7.67mg of MPEG2000-DSPE were weighed, heated to 70°C and dissolved in 18.18g of the aqueous phase prepared in (2), then added into 40g of the blank liposome prepared in (3) incubated at 70°C, and heated for loading drug for 10 minutes to obtain a drug-loaded liposome, which was dialyzed by a tangential flow ultrafiltration device to remove free gemcitabine and obtain a gemcitabine liposome. The obtained liposome had an average particle size of 80.20 nm, the concentration of gemcitabine hydrochloride was 0.85 mg/ml, the encapsulation efficiency was 98.17%, the ratio of total lipids to API was 35:1, and MPEG2000-DSPE accounts for 0.3% by mass of total lipids.

Example 8: Preparation of gemcitabine liposome

(1) Preparation of lipid phase

6.60g of hydrogenated soybean phosphatidylcholine, 2.12g of dipalmitoylphosphatidylglycerol, and 1.09g of cholesterol were weighed, heated to 60°C and dissolved in a mixed solvent of 60ml of anhydrous ethanol-6ml of water, and kept at the temperature for later use.

(2) Preparation of water phase

65.00g of ammonium sulfate, 23.00g of disodium hydrogen phosphate, and 5.25g of sodium dihydrogen phosphate (monohydrate) were weighed and dissolved in 2000g of water to prepare a 246 mM ammonium sulfate-100 mM phosphate buffer solution, which was kept at 60°C for later use.

(3) Preparation of blank liposome

Under the same temperature conditions, the lipid phase prepared in (1) was injected into 300 ml of the water phase prepared in (2) at a speed of 60 ml/min, continuously heated and stirred for 10 minutes to obtain a primary emulsion of liposome, and the primary emulsion of liposome obtained was subjected to extrusion to reach an average particle size of 65nm, the organic solvent was removed by dialysis using a tangential flow ultrafiltration device to obtain a blank liposome with an aqueous interior containing the 246mM ammonium sulfate-100 mM phosphate buffer solution.

(4) Preparation of drug-loaded liposomes

1.24g of gemcitabine hydrochloride and 0.167g of sodium hydroxide were weighed, dissolved in 17.45g of water by heating to 70°C, then added into 30g of the blank liposome prepared in (3) incubated at 70°C, and heated for loading drug for 10 minutes to obtain a drug-loaded liposome, which was diluted 5 times by adding 10% sucrose-0.155% histidine (w/v, pH 6.5), and then dialyzed using a tangential flow ultrafiltration device to remove free gemcitabine to obtain a gemcitabine liposome. The resulting liposome had an average particle size of 73.83nm, the concentration of gemcitabine hydrochloride was 0.75 mg/ml, the encapsulation efficiency was 98.39%, and the mass ratio of total lipids to API was 25:1.

Example 9: Preparation of gemcitabine liposome

(1) Preparation of lipid phase

13.72g of hydrogenated soybean phosphatidylcholine and 1.81g of cholesterol were weighed and dissolved in 100 ml of anhydrous ethanol by heating to 60°C, and kept at the temperature for later use.

(2) Preparation of aqueous phase

97.54g of ammonium sulfate, 68.99g of disodium hydrogen phosphate, and 15.74g of sodium dihydrogen phosphate (monohydrate) were weighed and dissolved in 3000g of water to prepare a 246 mM ammonium sulfate-200 mM phosphate buffer solution, which was kept at 60°C for later use.

(3) Preparation of blank liposome

Under the same temperature conditions, the lipid phase prepared in (1) was injected into 500ml of the water phase prepared in (2) at a speed of 60ml/min, continuously heated and stirred for 10 minutes to obtain a primary emulsion of liposome, and the primary emulsion of liposome obtained was subjected to extrusion to reach an average particle size of 72 nm, the organic solvent was removed by dialysis using a tangential flow ultrafiltration device to obtain 110g of a blank liposome containing the 246 mM ammonium sulfate-200 mM phosphate buffer solution as aqueous interior.

(4) Preparation of drug-loaded liposome

1.35g of gemcitabine hydrochloride, 0.18g of sodium hydroxide, and 36.0mg of MPEG2000-DSPE were weighed, heated to 70°C and dissolved in 26.82g of water, then added into 25g of the blank liposome prepared in (3) incubated at 70°C, and heated for 10 minutes to obtain a drug-loaded liposome, which was diluted 2 times by adding 10% sucrose-0.155% histidine (w/v, pH 6.5), and dialyzed using a tangential flow ultrafiltration device to remove free gemcitabine and obtain a gemcitabine liposome. The obtained liposome had an average particle size of 74.76 nm, the concentration of gemcitabine hydrochloride was 1.21 mg/ml, the encapsulation efficiency was 99.01%, the mass ratio of total lipids to API was 22:1, and MPEG2000-DSPE accounts for 1.6% by mass of total lipids.

Example 10: Preparation of gemcitabine liposome

(1) Preparation of lipid phase

13.73g of hydrogenated soybean phosphatidylcholine and 1.81g of cholesterol were weighed and dissolved in 100ml of anhydrous ethanol by heating to 60°C, and kept at the temperature for later use.

(2) Preparation of water phase

135.33g of ammonium sulfate was weighed and dissolved in 3000g of water to prepare a 341 mM ammonium sulfate solution, which was kept at 60°C for later use.

(3) Preparation of blank liposome

Under the same temperature conditions, the lipid phase prepared in (1) was injected into 537.17g of the water phase prepared in (2) at a speed of 100ml/min, continuously heated and stirred for 30 minutes to obtain a primary emulsion of liposome, and the primary emulsion of liposome obtained was subjected to extrusion to reach an average particle size of 71.86 nm, dialyzed using a tangential flow ultrafiltration device to remove the organic solvent and obtain 126g of a blank liposome containing the 341 mM ammonium sulfate solution as aqueous interior.

(4) Preparation of drug-loaded liposome

5.29g gemcitabine hydrochloride, 7.16g sodium hydroxide, and 0.8007g MPEG2000-DSPE were weighed and dissolved in 58.68g water by heating to 70°C, then added into 53g of the blank liposome prepared in (3) incubated at 70°C, and heated for loading drug for 10 minutes to obtain a drug-loaded liposome, which was diluted 5 times by adding 10% sucrose-0.155% histidine (w/v, pH6.5), then dialyzed using a tangential flow ultrafiltration device to remove free gemcitabine and obtain a gemcitabine liposome. The obtained liposome had an average particle size of 96.24 nm, the concentration of gemcitabine hydrochloride was 1.87 mg/ml, the encapsulation efficiency was 97.21%, the mass ratio of total lipids to API was 25:1, and MPEG2000-DSPE accounts for 12% by mass of total lipids.

Example 11: Preparation of gemcitabine liposome

(1) Preparation of lipid phase

8.26g of hydrogenated soybean phosphatidylcholine and 1.09g of cholesterol were weighed, dissolved in 60 ml of anhydrous ethanol by heating to 60°C, and kept at the temperature for later use.

(2) Preparation of water phase

112.0g of ammonium sulfate, 23.03g of disodium hydrogen phosphate, and 5.25g of sodium dihydrogen phosphate monohydrate were weighed and dissolved in 2000g of water to prepare a 424 mM ammonium sulfate-100 mM phosphate buffer solution, which was kept at 60°C for later use.

(3) Preparation of blank liposome

Under the same temperature conditions, the lipid phase prepared in (1) was injected into 308.5g of the water phase prepared in (2) at a speed of 60ml/min, continuously heated and stirred for 10 minutes to obtain a primary emulsion of liposome, and the primary emulsion of liposome obtained was subjected to extrusion to reach an average particle size of 76.41nm, dialyzed using a tangential flow ultrafiltration device to remove the organic solvent and obtain 61.5g of a blank liposome containing the 424 mM ammonium sulfate-100 mM phosphate solution as aqueous interior.

(4) Preparation of drug-loaded liposome

2.27g of gemcitabine hydrochloride, 0.31g of sodium hydroxide, and 0.337g of MPEG2000-DSPE were weighed and dissolved in 13.26g of the aqueous phase prepared in (2) by heating to 70°C, then added into 30g of the blank liposome prepared in (3) incubated at 70°C, and heated for loading drug for 10 minutes to obtain a drug-loaded liposome, which was diluted 5 times by adding 1.5-fold dilution of the aqueous phase prepared in (2), dialyzed using a tangential flow ultrafiltration device to remove free gemcitabine and obtain a gemcitabine liposome. The obtained liposome had an average particle size of 85.86nm, the concentration of gemcitabine hydrochloride was 1.68 mg/ml, the encapsulation efficiency was 98.83%, the mass ratio of total lipids to API was 15:1, MPEG2000-DSPE accounts for 12.5% by mass of total lipids.

Example 12: Preparation of gemcitabine liposome

(1) Preparation of lipid phase

6.87g of hydrogenated soybean phosphatidylcholine and 0.90g of cholesterol were weighed, dissolved in 50ml of anhydrous ethanol by heating to 60°C, and kept at the temperature for later use.

(2) Preparation of water phase

275g of glucose, 28.75g of disodium hydrogen phosphate, and 6.56g of sodium dihydrogen phosphate monohydrate were weighed and dissolved in 2500g of water to prepare a 610 mM glucose-100 mM phosphate solution, which was kept at 60°C for later use.

(3) Preparation of blank liposome

Under the same temperature conditions, the lipid phase prepared in (1) was injected into 258g of the water phase prepared in (2) at a speed of 60ml/min, continuously heated and stirred for 10 minutes to obtain a primary emulsion of liposome, and the primary emulsion of liposome obtained was subjected to extrusion to reach an average particle size of 69.03nm, dialyzed using a tangential flow ultrafiltration device to remove the organic solvent and obtain 57.5g of a blank liposome containing the 610 mM glucose-100 mM phosphate solution as aqueous interior.

(4) Preparation of drug-loaded liposome

1.73g of gemcitabine hydrochloride, 0.23g of sodium hydroxide, and 0.257g of MPEG2000-DSPE were weighed and dissolved in 16.9g of the aqueous phase prepared in (2) by heating to 70°C, then added into 20g of the blank liposome prepared in (3) incubated at 70°C, and heated for loading drug for 10 minutes to obtain a drug-loaded liposome, which was diluted 5 times by adding 1.5-fold dilution of the aqueous phase prepared in (2), and dialyzed using a tangential flow ultrafiltration device to remove free gemcitabine and obtain a gemcitabine liposome. The obtained liposome had an average particle size of 81.9nm, the concentration of gemcitabine hydrochloride was 1.70 mg/ml, the encapsulation efficiency was 94.32%, the mass ratio of total lipids to API was 16:1, MPEG2000-DSPE accounts for 12.5% by mass of total lipids.

Example 13: Preparation of gemcitabine liposome (Comparative Example, using mannitol-phosphate buffer solution as aqueous interior)

(1) Preparation of lipid phase

6.87g of hydrogenated soybean phosphatidylcholine and 0.90g of cholesterol were weighed, dissolved in 50ml of anhydrous ethanol by heating to 60°C, and kept at the temperature for later use.

(2) Preparation of water phase

220g of mannitol, 23.00g of disodium hydrogen phosphate, and 5.24g of sodium dihydrogen phosphate monohydrate were weighed and dissolved in 2000g of water to prepare a 604 mM mannitol-100 mM phosphate solution, which was kept at 60°C for later use.

(3) Preparation of blank liposome

Under the same temperature conditions, the lipid phase prepared in (1) was injected into 258.5g of the water phase prepared in (2) at a speed of 60ml/min, continuously heated and stirred for 10 minutes to obtain a primary emulsion of liposome, and the primary emulsion of liposome obtained was subjected to extrusion to reach an average particle size of 71.68nm, and dialyzed using a tangential flow ultrafiltration device to remove the organic solvent and obtain 52.0g of a blank liposome containing the 604 mM mannitol-100 mM phosphate solution as aqueous interior.

(4) Preparation of drug-loaded liposome

1.52g of gemcitabine hydrochloride, 0.27g of sodium hydroxide, and 0.230g of MPEG2000-DSPE were weighed, heated to 70°C and dissolved in 12.4g of the aqueous phase prepared in (2), then added into 20g of the blank liposome prepared in (3) incubated at 70°C, and heated for loading drug for 10 minutes to obtain a drug-loaded liposome, which was diluted 5 times by adding 1.5-fold dilution of the aqueous phase prepared in (2), and dialyzed using a tangential flow ultrafiltration device to remove free gemcitabine and obtain a gemcitabine liposome. The obtained liposome had an average particle size of 80.49nm, the concentration of gemcitabine hydrochloride was 0.79 mg/ml, the encapsulation efficiency was 98.60%, the mass ratio of total lipids to API was 25:1, and MPEG2000-DSPE accounts for 12.5% by mass of total lipids.

Example 14: Preparation of gemcitabine liposome

(1) Preparation of lipid phase

8.23g of hydrogenated soybean phosphatidylcholine, 1.08g of cholesterol, and 1.30g of MPEG2000-DSPE were weighed, and dissolved in 60 ml of anhydrous ethanol by heating to 60°C, and kept at the temperature for later use.

(2) Preparation of water phase

13.8g of gemcitabine hydrochloride and 1.85g of sodium hydroxide were weighed and dissolved in 300g of ammonium sulfate solution (340 mM, obtained by dissolving 13.5g of ammonium sulfate in 300g of water), and kept at 60°C for later use.

(3) Preparation of drug-loaded liposome

Under the same temperature conditions, with a total flow rate of 36ml/min and an oil-to-water ratio of 1:5, the lipid phase was injected into the water phase to obtain a primary emulsion of drug-loaded liposome, which was extruded through micro-jet high-pressure homogenizer to reach a particle size of 90 nm, then diluted 5 times by adding 10% sucrose-0.155% histidine (w/v, pH6.5) solution, and subjected to ultrafiltration, dialysis and concentration by using a tangential flow ultrafiltration device to remove free drug and ethanol (the dialysate was a pH6.5 10% sucrose-0.155% histidine solution). After the dialysis was completed, a gemcitabine liposome was obtained. The liposome obtained had an average particle size of 95.23nm, the concentration of gemcitabine hydrochloride was 1.21 mg/ml, the encapsulation efficiency was 95.98%, the mass ratio of total lipids to API was 23:1, and MPEG2000-DSPE accounts for 12.3% by mass of total lipids.

Experimental Example 1: Stability investigation of gemcitabine liposomes

The gemcitabine liposomes prepared in Examples 5, 8, and 9 were placed in a refrigerator at 2 to 8°C for 0 days and 150 days, respectively, and the changes in encapsulation efficiency and content were investigated. As shown in Table 1 below, the changes in encapsulation efficiency and content of the drug-loaded liposomes were less than 5% within 150 days, indicating that the prepared gemcitabine liposomes had good stability.

Table 1: Gemcitabine liposome encapsulation efficiency stability test

| Name | Days | | | |
|---|---|---|---|---|
| | Day 0 | | Day 150 | |
| | Content (mg/ml) | Encapsulation efficiency (%) | Content (mg/ml) | Encapsulation efficiency (%) |
| Example 5 | 0.94 | 98.35 | 0.90 | 97.43 |
| Example 8 | 0.75 | 98.39 | 0.77 | 96.63 |
| Example 9 | 1.21 | 99.01 | 1.23 | 98.58 |

Experimental Example 2: Pharmacokinetic tests of gemcitabine conventional injection and gemcitabine liposomes

Mouse species: Balb/c female tumor-bearing mice.

Grouping: Conventional injection group, liposome groups, 4 animals in each group, 4 groups in total, and 16 animals in total.

Sample preparation: Conventional injection (GEM): gemcitabine for injection (0.2g/bottle, Zefei (Gemcitabine Hydrochloride Injection)), which was diluted by adding 5ml of 0.9% normal saline so that the gemcitabine concentration was 38 mg/ml. Liposome groups: the methods for preparing the liposomes were detailed in Example 5, Example 6 and Example 7.

Blood collection point design and blood collection method: Liposome groups: 0.15 ml of blood (anticoagulated with EDTA EDTAEDTA-K2) was taken from orbital vein at 0.083h, 0.5h, 1h, 2h, 4h, 8h, 24h, 48h and 72h after administration. Conventional injection group: 0.15ml of blood (anticoagulated with EDTA) was taken from orbital vein at 0.5h, 1h, 3h, 8h, 24h and 48h after administration.

Dosage: Conventional injection: 200mg/kg; liposome groups: 4mg/kg (i.e., 4mpk).

Blood sample processing: The collected whole blood was immediately stored in crushed ice and centrifuged at 4000 rpm for 10 minutes within 1 hour to separate plasma (4°C). The collected plasma was stored in a -80°C refrigerator until testing.

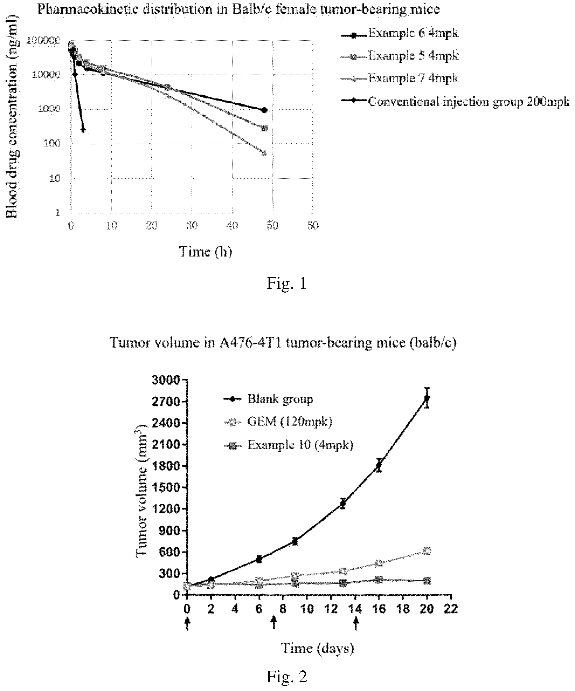

Results: The pharmacokinetic results were shown in Fig. 1. Compared with the gemcitabine conventional injection, the gemcitabine liposome groups all showed significantly prolonged blood half-life of gemcitabine. The conventional injection group showed that the amount of gemcitabine after 8h was lower than limit of detection, while the liposome groups showed that gemcitabine was stilled detectable in blood after 48 hours.

Experimental Example 3: Pharmacodynamic study of gemcitabine conventional injection and liposome in 4T1 tumor-bearing mice

Mouse species: Balb/c mouse.

Grouping: vehicle group (5% glucose injection), gemcitabine conventional injection group (GEM), liposome group (Example 10), 6 animals in each group, 3 groups in total, and a total of 18 animals were needed.

Sample preparation: Conventional injection: gemcitabine for injection (0.2g/bottle, Zefei (Gemcitabine Hydrochloride Injection)), which was diluted by adding 5ml of 0.9% normal saline so that the gemcitabine concentration was 38 mg/ml. Liposome group: The preparation method of the liposome was detailed in Example 10.

Cell culture

4T1 cells were cultured in monolayer in vitro. The culture conditions comprised: RPMI 1640 medium plus 10% fetal calf serum, and culturing at 37°C and 5% $CO_2$ in an incubator. Trypsin-EDTA was used twice/week for digestion and passage. When the cells were in the exponential growth phase, the culture medium was taken for mycoplasma detection, and the cells were collected and counted.

Inoculation, grouping and administration

Before inoculation of tumor cells, the hair of the test mice at the inoculation site was removed with an electric shaver, and the animals were numbered sequentially with mouse-specific numbering earrings. Each mouse was inoculated subcutaneously with $2 \times 10^5$ 4T1 cells (suspended in 0.1 ml of PBS) on the right scapular. When the average volume of tumor in mice grew to about 100 to 150 $mm^3$, mice with tumors that were too small or too large were eliminated. The remaining mice were randomly divided into groups based on tumor volume and animal weight and given medication. Blank group: 0.9% normal saline; gemcitabine conventional injection (GEM): 120 mg/kg (i.e., 120 mpk); liposome group (Example 10): 4 mg/kg (i.e., 4 mpk); administered once per week for 3 consecutive weeks; the maximum diameter and shortest diameter of tumor were measured using a vernier caliper, and changes in tumor volume were observed and recorded. Wherein, tumor volume = maximum diameter $\times$ shortest diameter $\times$ shortest diameter $\div$ 2

Results: As shown in Fig. 2, at a very low dose (4mpk, 1/30 of that of the conventional injection group), the gemcitabine liposome group had higher efficacy than the conventional injection group (120mpk), indicating that the gemcitabine liposome had potential synergistic advantage.

Experimental Example 4: Pharmacodynamic study of gemcitabine conventional injection (GEM group) and gemcitabine liposome groups (Example 1 and Example 6) in BxPC-3 tumor-bearing mice

Mouse species: Balb/c nu mouse.

Grouping: vehicle group (5% glucose injection), gemcitabine conventional injection group (GEM), liposome groups (Example 1 and Example 6), 6 animals in each group, 5 groups in total, and a total of 30 animals were needed.

Sample preparation: Conventional injection: gemcitabine for injection (0.2g/bottle, Zefei (Gemcitabine Hydrochloride Injection)), which was diluted by adding 5ml of 0.9% normal saline so that the gemcitabine concentration was 38 mg/ml. Liposome groups: The preparation methods of the liposomes were detailed in Example 1 and Example 6.

Cell culture

BxPC-3 cells were cultured in monolayer in vitro at 37°C and 5% $CO_2$ in an incubator. Trypsin-EDTA was used once or twice/week for digestion and passage. When the cells were in the exponential growth phase, the culture medium was taken for mycoplasma detection, and the cells were collected and counted.

Inoculation, grouping and administration

Each mouse was inoculated subcutaneously with $5 \times 10^6$ BxPC-3 cells (suspended in 0.1 ml PBS) on the right scapular. When the average tumor volume grew to about 200 mm$^3$, mice with tumors that were too small or too large were eliminated, and the remaining mice were randomly divided into groups according to tumor volume and animal weight and given medication. Vehicle group: 0.9% sodium chloride solution; conventional injection: 240 mg/kg; liposome groups: Example 1: 4 mg/kg; Example 6: 1 mg/kg and 4 mg/kg; administered once per week for 3 consecutive weeks; the maximum diameter and shortest diameter of tumor were measured using a vernier caliper, and changes in tumor volume and mouse body weight were observed and recorded. Wherein, tumor volume = maximum diameter × shortest diameter × shortest diameter ÷ 2

$$\text{Tumor inhibition rate TGI (\%)}_{\text{(tumor volume)}} = [1 - (T_{Vt} - T_{V0}) / (C_{Vt} - C_{V0})] \times 100\%$$

wherein,

$T_{V0}$ represented the average tumor volume of the test group when grouping and administering;
$T_{V\epsilon}$ represented the average tumor volume of the test group on day t after administration;
$C_{V0}$ represented the average tumor volume of the vehicle group when grouping and administering;
$C_{Vt}$ represented the average tumor volume of the vehicle group on day t after administration.

When the tumor regressed, TGI (%) $_{\text{(tumor volume)}}$ = 100% - $(T_{V\epsilon} - T_{V0})/T_{V0} \times 100\%$.

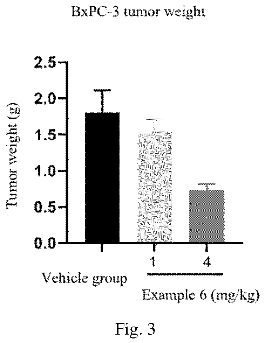

Results: The gemcitabine conventional injection group (GEM, 240 mg/kg) and liposome group-Example 1 at dose of 4 mg/kg showed intolerance, hunched back and diarrhea were observed, death occurred starting on day 4 after the first administration, 4/6 in the GEM group died on day 6 after administration, all animals died (6/6) in the liposome group-Example 1 (4 mg/kg), and no death occurred in the liposome group-Example 6. After 6 weeks of continuous administration, the liposome group-Example 6 at 4 mg/kg showed a TGI of 69.74% in gemcitabine-insensitive tumors. Compared with the vehicle group, p<0.01, which was a statistically significant difference. It showed that the liposome of Example 6 exhibited anti-tumor efficacy and high tolerance (Fig. 3).

Experimental Example 5: Impact of buffer salt system in aqueous interior on the generation of API impurities
Method: The gemcitabine liposomes prepared in Example 1, Example 4, Example 11, Example 12, and Example 13 were placed at 2 to 8°C and 25°C, respectively, and the impact of buffer salt system in aqueous interior on the generation of impurities was investigated on day 0, day 10, and day 30. The results were shown in Table 2 below:
Results: Compared with the glucose-phosphate and mannitol-phosphate buffer systems, the sulfate-phosphate buffer system produced less impurities. Especially after being placed at room temperature, API in the liposome containing the sulfate buffer system had better stability.

Table 2: Impact of different buffer salt systems in aqueous interior on the generation of API impurities

| Sample name | Temperature (°C) | Days | API impurities | |
|---|---|---|---|---|
| | | | α-isomer (%) | Other impurities (%) |
| Example 1 | 0 | 0 | 0.212 | 0.059 |
| | 2~8 | 10 | 1.339 | ND |
| | 25 | | 9.882 | 0.292 |
| | 2~8 | 30 | 2.347 | 0.099 |
| | 25 | | 17.709 | 0.418 |
| Example 4 | 0 | 0 | ND | 0.033 |
| | 2~8 | 10 | ND | 0.076 |
| | 25 | | ND | 1.468 |
| | 2~8 | 30 | ND | 0.03 |
| | 25 | | ND | 1.591 |
| Example 11 | 0 | 0 | ND | 0.060 |
| | 2~8 | 10 | ND | 0.11 |
| | 25 | | ND | 0.123 |
| | 2~8 | 30 | ND | 0.053 |
| | 25 | | ND | 0.069 |
| Example 12 | 0 | 0 | ND | 0.077 |
| | 2~8 | 10 | ND | 0.149 |
| | 25 | | ND | 4.732 |
| | 2~8 | 30 | ND | 0.093 |
| | 25 | | ND | 6.777 |
| Example 13 | 0 | 0 | ND | ND |
| | 2~8 | 10 | ND | ND |
| | 25 | | ND | 0.093 |
| | 2~8 | 30 | ND | 0.051 |
| | 25 | | ND | 3.112 |
| "ND" means not detected. | | | | |

Although the specific embodiments of the present invention have been described in detail, those skilled in the art will understand that various modifications and substitutions can be made to those details based on all teachings that have been disclosed, and these changes are within the scope of the present invention. The full scope of the present invention is given by the appended claims and any equivalents thereof.

## Claims

1. A gemcitabine liposome, which comprises a liposome membrane and an aqueous interior encapsulated by the liposome membrane, wherein,

the components constituting the liposome membrane comprise a phospholipid, cholesterol and optionally a long-circulating functional material; the aqueous interior comprises gemcitabine or a pharmaceutically acceptable salt thereof, and a salt solution; and,
the weight ratio of the phospholipid to cholesterol is 2:1 to 20:1;
the long-circulating functional material accounts for 0% to 30% by mass of total lipids;

the weight ratio of total lipids to gemcitabine or the pharmaceutically acceptable salt thereof in the liposome is 1:1 to 100:1;

the molar concentration of the salt solution is 50 to 800 mM.

2. The gemcitabine liposome according to claim 1, **characterized by** one or more items of the following:

(1) the phospholipid is one or more selected from the group consisting of lecithin, cephalin, soybean lecithin, phosphatidylethanolamine, phosphatidylcholine, phosphatidylglycerol, phosphatidylserine, sphingomyelin, cardiolipin and derivatives thereof (including hydrogenated phospholipids obtained by hydrogenating the above phospholipids);

(2) when there contained one type of phospholipids, it is the one selected from the group consisting of a neutral phospholipid, a negatively charged phospholipid and sphingomyelin (SM), preferably phosphatidylcholine or sphingomyelin, such as hydrogenated soybean phosphatidylcholine (HSPC), distearoylphosphatidylcholine (DSPC), dipalmitoylphosphatidylcholine (DPPC), sphingomyelin, hydrogenated sphingomyelin (HSM); when there contained two types of phospholipids, it is a combination of a neutral phospholipid and a negatively charged phospholipid, preferably a combination of a phosphatidylcholine and a phosphatidylglycerol, such as DSPC and distearoylphosphatidylglycerol (DSPG), HSPC and DSPG, or HSPC and dipalmitoylphosphatidylglycerol (DPPG);

(3) the long-circulating functional material is one or more selected from polyethylene glycol (PEG), polyethylene glycol-vitamin E succinate (TPGS), polyethylene glycol-cholesterol (PEG-CHOL), polyethylene glycol-modified distearoylphosphatidylethanolamine (MPEG-DSPE), polyethylene glycol-modified dimyristoylphosphatidylethanolamine (MPEG-DMPE), polyethylene glycol-modified dipalmitoylphosphatidylethanolamine (MPEG-DPPE), and polyethylene glycol-modified lipid, preferably a phospholipid which is modified by polyethylene glycol and is the same type as the phospholipid that constitutes the liposome membrane; preferably, the polyethylene glycol has a molecular weight of 500 Dalton to 10,000 Daltons, more preferably 2000 to 5000 Daltons;

(4) the salt solution is one or more selected from the group consisting of a sodium salt solution, a potassium salt solution and an ammonium salt solution, and the acid radical ion contained in the salt solution is selected from the group consisting of sulfate radical, chloride ion, phosphate radical, hydrogen phosphate radical, dihydrogen phosphate radical, sucrose octasulfate radical, citrate radical, gluconate radical, methanesulfonate radical, acetate radical, oxalate radical and 4-hydroxyethyl piperazine ethanesulfonate radical, preferably one or more selected from the group consisting of sulfate radical, chloride ion, hydrogen phosphate radical, dihydrogen phosphate radical, sucrose octasulfate radical and acetate radical, more preferably sulfate radical or a combination of sulfate radical, chloride ion, phosphate radical, hydrogen phosphate radical and dihydrogen phosphate radical; more preferably, the salt solution is an ammonium sulfate solution, a solution of a combination of sodium sulfate and a salt of phosphate buffer, a solution of a combination of sodium chloride and a salt of phosphate buffer, or solution of a combination of ammonium sulfate and a salt of phosphate buffer;

(5) the weight ratio of the phospholipid to cholesterol is 2:1 to 15:1, preferably 2:1 to 10:1, preferably 2.5:1 to 10:1;

(6) the long-circulating functional material accounts for 0% to 20% by mass of total lipids;

(7) when the phospholipid is sphingomyelin or a negatively charged phospholipid, the liposome membrane does not contain the long-circulating functional material;

(8) the weight ratio of total lipids to gemcitabine or the pharmaceutically acceptable salt thereof in the liposome is 5:1 to 80:1; preferably 10:1 to 50:1;

(9) the molar concentration of the salt solution in the aqueous interior is 100 to 800 mM, preferably 200 to 600 mM.

3. The gemcitabine liposome according to claim 1 or 2, **characterized by** one or more items of the following:

1) the gemcitabine liposome has an average particle size of 10 to 200 nm, preferably 30 to 150 nm, and more preferably 50 to 100 nm;

2) the concentration of gemcitabine or the pharmaceutically acceptable salt thereof in the liposome is 0.1 to 3 mg/ml, preferably 0.1 to 2 mg/ml;

3) the encapsulation efficiency is more than 85%, such as more than 95%, more than 96%, more than 97%, more than 98%, more than 99%, more than 99.5%.

4. A method for preparing the gemcitabine liposome according to any one of claims 1 to 3, which comprises the following steps:

S 1, preparation of an aqueous phase: dissolving gemcitabine or a pharmaceutically acceptable salt thereof in a salt solution with a concentration of 50 to 800 mM to obtain the aqueous phase;

S2, preparation of a lipid phase: dissolving phospholipid and cholesterol in a weight ratio of 2:1 to 20:1 in a solvent to obtain the lipid phase, wherein the solvent is one or more selected from the group consisting of ethanol, methanol, ethyl acetate, chloroform, dichloromethane and diethyl ether;

S3, preparation of a drug-loaded liposome: mixing the aqueous phase obtained in S1 and the lipid phase obtained in S2 by organic solvent injection method, thin-film hydration method, reverse-phase evaporation method, emulsification method, freeze-thaw cycles, pipeline mixing method, or microfluidic method to obtain a primary emulsion of liposome containing gemcitabine or the pharmaceutically acceptable salt thereof and a salt solution of an aqueous interior;

S4, granulation: granulating the primary emulsion of the drug-loaded liposome by using one or more selected from the group consisting of shearer, homogenizer and extruder to reach a target particle size;

S5, removal of free drugs: removing free drugs by one or more treatments selected from filtration, centrifugation, dialysis, ultrafiltration, concentration, and etc. to obtain the drug-loaded liposome comprising gemcitabine or the pharmaceutically acceptable salt thereof;

optionally, adding a long-circulating functional material when preparing the lipid phase in step S2.

5. The method of claim 4, **characterized by** one or more items of the following items:

(A) the salt solution in step S1 is an ammonium sulfate solution; preferably, the molar concentration of the ammonium sulfate solution is 50 to 800 mM, preferably 50 to 600 mM, preferably 50 to 400 mM, preferably 50 to 200 mM, preferably 50 to 100 mM;

(B) the phospholipid in step S2 is HSPC;

(C) the long-circulating functional material in step S2 is MPEG2000-DSPE;

(D) in step S2, the weight ratio of the phospholipid to cholesterol is 2:1 to 15:1, preferably 2:1 to 10:1, preferably 2.5:1 to 10:1, more preferably 5:1 to 8:1;

(E) in step S2, the long-circulating functional material added accounts for 0% to 30%, preferably 0% to 20%, preferably 10% to 20%, by mass of total lipids;

(F) in step S3, at a lipid-to-water ratio of 1:1 to 1:20, mixing the aqueous phase prepared in S1 and the lipid phase prepared in S2 to obtain the drug-loaded liposome; preferably, the lipid-to-water ratio is 1:3, 1:5, 1:7, 1:10 or 1:20;

(G) conducting step S3 at or above the phase transition temperature of the lipid; preferably, at 30°C to 70°C (e.g., 30°C to 40°C, 50°C to 60°C, 60°C to 70°C, or 60°C to 65°C);

(H) in step S4, granulating the primary emulsion of the drug-loaded liposome to reach an average particle size of 10 to 200 nm, such as 30 to 200 nm, 50 to 200 nm, 150 to 200 nm, 10 to 150 nm, 30 to 150 nm, 50 to 150 nm, 100 to 150 nm, 10 to 100 nm, 30 to 100 nm, 50 to 100 nm, 10 to 50 nm, 30 to 50 nm, or 10 to 30 nm, preferably 50 to 100 nm.

6. A method for preparing the gemcitabine liposome according to any one of claims 1 to 3, which comprises the following steps:

SI, preparation of an aqueous phase: preparing a salt solution having a concentration of 50 to 800 mM as the aqueous phase;

SII, preparation of a lipid phase: dissolving a phospholipid and cholesterol in a weight ratio of 2:1 to 20:1 in a solvent to obtain the lipid phase, wherein the solvent is one or more selected from the group consisting of water, ethanol, methanol, ethyl acetate, chloroform, dichloromethane and diethyl ether;

SIII, preparation of a primary emulsion of blank liposome: mixing the aqueous phase obtained in SI and the lipid phase obtained in SII in appropriate amounts by organic solvent injection method, thin-film hydration method, reverse-phase evaporation method, emulsification method, freeze-thaw cycles, pipeline mixing method, or microfluidic method to obtain the primary emulsion of blank liposome;

SIV, granulation: granulating the primary emulsion of blank liposome by one or more selected from the group consisting of shear, homogenizer and extruder to reach a target particle size;

SV, removal of organic solvent: removing the organic solvent by one or more treatments selected from the group consisting of filtration, centrifugation, dialysis, ultrafiltration, concentration, and etc. to obtain a blank liposome;

SVI, preparation of an API solution: dissolving gemcitabine or a pharmaceutically acceptable salt thereof in water or the aqueous phase of step SI which is diluted or undiluted to prepare the API solution;

SVII, preparation of a drug-loaded liposome: heating and incubating the obtained blank liposome and the API solution at 30°C to 75°C for 5 to 60 minutes to obtain the drug-loaded liposome;

SVIII, removal of free drugs: removing the free drugs by one or more treatments selected from filtration, centrifugation, dialysis, ultrafiltration, concentration, and etc. to obtain the drug-loaded liposome containing gemcitabine or the pharmaceutically acceptable salt thereof;
optionally, adding a long-circulating functional material when preparing the API solution in step SVI or preparing the drug-loaded liposome in step SVII.

7. The method according to claim 6, **characterized by** one or more items of the following:

(a) the salt solution in step SI is an ammonium sulfate solution, a mixed solution of sodium sulfate and a salt of phosphate buffer, a mixed solution of sodium chloride and a salt of phosphate buffer, or a mixed solution of ammonium sulfate and a salt of phosphate buffer, preferably, the concentration of the ammonium sulfate solution is 100 to 800 mM, preferably 200 to 600 mM, more preferably 200 to 400 mM, more preferably 300 to 400 mM, preferably, the concentration of sodium sulfate, sodium chloride or ammonium sulfate in the mixed solution is 100 to 800 mM (preferably 200 to 600 mM, more preferably 200 to 500 mM, more preferably 250 to 500 mM), and the concentration of the salt of phosphate buffer is 100 to 200 mM;
(b) the phospholipid in step SII is selected from the group consisting of sphingomyelin, hydrogenated soybean phosphatidylcholine, and a combination of hydrogenated soybean phosphatidylcholine and dipalmitoylphosphatidylglycerol, preferably, the weight ratio of the phospholipid to cholesterol in step SII is 2:1 to 15:1, preferably 2:1 to 10:1, more preferably 2:1 to 8:1, such as 2:1 to 3:1 or 7:1 to 8:1;
(c) the solvent in step SII is ethanol or a mixed solution of ethanol and water;
(d) in step SIII, adding the lipid phase prepared in step SII to an appropriate amount of the aqueous phase prepared in step SI at a lipid-to-water ratio of 1:1 to 1:20 (e.g., 1:3, 1:5, 1:10, 1:20), stirring at the phase transition temperature of the lipid or above the phase transition temperature of the lipid to obtain the primary emulsion of blank liposome;
(e) the long-circulating functional material is MPEG2000-DSPE.

8. The method according to claim 6 or 7, **characterized by** one or more items of the following:

(i) step SIII comprises the following steps: stirring and emulsifying at 30°C to 75°C (e.g., 30°C to 40°C, 50°C to 60°C, or 60°C to 70°C) to obtain the primary emulsion of blank liposome;
(ii) the primary emulsion of blank liposome prepared in step SIV has an average particle size of 10 to 200 nm, such as 30 to 200 nm, 50 to 150 nm, 50 to 100 nm, 60 to 80 nm, specifically, such as 60 nm, 65nm, 70 nm, 75nm, or 80 nm;
(iii) in step SVII, the weight ratio of gemcitabine or the pharmaceutically acceptable salt thereof to total lipids in the blank liposome is 2:1 to 1:10, preferably 2:1 to 1:5, for example 1:1 to 1:5, for another example 1:1 to 1:3, for another example 1:1 to 1:2; specifically, such as 1:1, 1:1.1, 1:1.2, 1:1.3, 1:1.4, 1:1.5, 1:1.6, 1:1.7, 1:1.8, 1:1.9, 1:2.0;
(iv) the incubation temperature in step SVII is 50°C to 75°C (for example, 65°C to 70°C);
(v) the incubation time in step SVII is 10min to 45min;
(vi) the mass fraction of the long-circulating functional material added in step SVI or SVII in the liposome is 0% to 15%, for example, 0% or 0.01% to 15%, specifically 0.01%, 0.05%, 1%, 2%, 3%, 4%, 5%, 6%, 7%, 8%, 9%, 10%, 11%, 12%, 13%, 14% or 15%.

9. A liposome preparation, which comprises the gemcitabine liposome according to any one of claims 1 to 3 or the gemcitabine liposome prepared by the method according to any one of claims 4 to 8, and one or more pharmaceutically acceptable carrier; preferably, the liposome preparation comprises an osmotic pressure regulator, such as a sucrose-histidine solution, preferably a 10% sucrose-histidine solution, preferably a 10% sucrose-0.155% histidine solution;
preferably, the liposome preparation is an injection.

10. Use of the gemcitabine liposome according to any one of claims 1 to 3 or the liposome preparation according to claim 9 in the manufacture of an anti-tumor drug; preferably, the tumor is selected from the group consisting of pancreatic cancer, breast cancer, non-small cell lung cancer and ovarian cancer.

11. A method for treating a tumor, which comprises a step of administering to a subject in need thereof with an effective amount of the gemcitabine liposome according to any one of claims 1 to 3 or the liposome preparation according to claim 9; preferably, the tumor is selected from the group consisting of pancreatic cancer, breast cancer, non-small cell lung cancer and ovarian cancer.

Fig. 1

Fig. 2

BxPC-3 tumor weight

Fig. 3

<div align="center">**INTERNATIONAL SEARCH REPORT**</div>

| International application No. |
| --- |
| **PCT/CN2023/081594** |

| A. | CLASSIFICATION OF SUBJECT MATTER |
| --- | --- |

A61K9/127(2006.01)i; A61K31/7068(2006.01)i; A61K47/24(2006.01)i; A61K47/28(2006.01)i; A61K47/02(2006.01)i; A61P35/00(2006.01)i

According to International Patent Classification (IPC) or to both national classification and IPC

| B. | FIELDS SEARCHED |
| --- | --- |

Minimum documentation searched (classification system followed by classification symbols)

IPC: A61K A61P

Documentation searched other than minimum documentation to the extent that such documents are included in the fields searched

Electronic data base consulted during the international search (name of data base and, where practicable, search terms used)

CNABS, CNTXT, CNKI, DWPI, VEN, PUBMED, STN, 科伦药物研究院, 郝斐, 苏正兴, 李明, 赵栋, 刘思川, 张瑞霞, 刘厦, 焦健, 吉西他滨, 吉西他宾, 脂质体, 磷脂, 胆固醇, 长循环, 盐, gemcitabine, liposome, phospholipid, phosphatide, cholesterol, long circulating, salt, 95058-81-4

| C. | DOCUMENTS CONSIDERED TO BE RELEVANT |
| --- | --- |

| Category* | Citation of document, with indication, where appropriate, of the relevant passages | Relevant to claim No. |
| --- | --- | --- |
| PX | CN 114306240 A (SICHUAN KELUN DRUG RESEARCH INSTITUTE CO., LTD.) 12 April 2022 (2022-04-12) <br> see embodiments 1-12, description paragraph [0182] | 1-11 |
| X | CN 111035616 A (SHANGHAI JINGFENG PHARMACEUTICAL CO., LTD.) 21 April 2020 (2020-04-21) <br> see embodiments 1-5, description, paragraphs [0002], [0018]-[0019], [0026], [0031], table 1 | 1-11 |
| X | CN 108348538 A (FUJIFILM CORPORATION) 31 July 2018 (2018-07-31) <br> see description, paragraphs [0083]-[0185], [0201]-[0212] | 1-11 |
| X | CN 106474067 A (SICHUAN INDUSTRIAL INSTITUTE OF ANTIBIOTICS, CHINA NATIONAL PHARMACEUTICAL GROUP CORPORATION et al.) 08 March 2017 (2017-03-08) <br> see embodiments 3-11, description, paragraph [0078] | 1-11 |
| X | WO 2005021012 A1 (TERUMO CORPORATION) 10 March 2005 (2005-03-10) <br> see embodiment 1 | 1-11 |

☑ Further documents are listed in the continuation of Box C.     ☑ See patent family annex.

| * | Special categories of cited documents: |
| --- | --- |
| "A" | document defining the general state of the art which is not considered to be of particular relevance |
| "D" | document cited by the applicant in the international application |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
| --- | --- |
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
| --- | --- |
| 6.6月 2023 (06.06.2023) | 15 June 2023 |

| Name and mailing address of the ISA/CN | Authorized officer |
| --- | --- |
| China National Intellectual Property Administration (ISA/CN) <br> China No. 6, Xitucheng Road, Jimenqiao, Haidian District, Beijing 100088 | |
| | Telephone No. |

Form PCT/ISA/210 (second sheet) (July 2022)

**INTERNATIONAL SEARCH REPORT**

| International application No. |
| --- |
| **PCT/CN2023/081594** |

**C. DOCUMENTS CONSIDERED TO BE RELEVANT**

| Category* | Citation of document, with indication, where appropriate, of the relevant passages | Relevant to claim No. |
| --- | --- | --- |
| X | US 2005249795 A1 (NEOPHARM. INC.) 10 November 2005 (2005-11-10) <br> see embodiments 1-2, description paragraphs [0012]-[0027], [0034] | 1-11 |
| X | 郑钦 等 (ZHENG, Qin et al.). "盐酸吉西他滨隐形脂质体的制备及工艺优化 (Preparation and Process Optimization of Gemcitabine Hydrochloride Stealth Liposomes)" <br> 中国抗生素杂志 (Chinese Journal of Antibiotics), <br> Vol. 42, No. (5), 31 May 2017 (2017-05-31), pages 382-388 <br> see entire document | 1-11 |
| X | XU, H.T. et al. "Development of Long-Circulating pH-Sensitive Liposomes to Circumvent Gemcitabine Resistance in Pancreatic Cancer Cells" <br> Pharm. Res., 06 February 2015 (2015-02-06), pp. 1-10 <br> see entire document | 1-11 |

Form PCT/ISA/210 (second sheet) (July 2022)

**INTERNATIONAL SEARCH REPORT**

International application No.

**PCT/CN2023/081594**

| Box No. II | Observations where certain claims were found unsearchable (Continuation of item 2 of first sheet) |
|---|---|

This international search report has not been established in respect of certain claims under Article 17(2)(a) for the following reasons:

1. ☑ Claims Nos.: **11**
   because they relate to subject matter not required to be searched by this Authority, namely:

   The subject matter of claim 11 relates to a method for treating a tumor, which does not comply with PCT Rule 39.1(iv). The present search report is provided on the basis that claim 11 is amended to be the same as claim 10.

2. ☐ Claims Nos.:
   because they relate to parts of the international application that do not comply with the prescribed requirements to such an extent that no meaningful international search can be carried out, specifically:

3. ☐ Claims Nos.:
   because they are dependent claims and are not drafted in accordance with the second and third sentences of Rule 6.4(a).

Form PCT/ISA/210 (continuation of first sheet) (July 2022)

## INTERNATIONAL SEARCH REPORT
### Information on patent family members

International application No.

**PCT/CN2023/081594**

| Patent document cited in search report | | | Publication date (day/month/year) | Patent family member(s) | | | Publication date (day/month/year) |
|---|---|---|---|---|---|---|---|
| CN | 114306240 | A | 12 April 2022 | None | | | |
| CN | 111035616 | A | 21 April 2020 | None | | | |
| CN | 108348538 | A | 31 July 2018 | US | 2018243214 | A1 | 30 August 2018 |
| | | | | US | 11166913 | B2 | 09 November 2021 |
| | | | | RU | 2020137385 | A | 03 December 2020 |
| | | | | RU | 2020137385 | A3 | 31 March 2021 |
| | | | | RU | 2768178 | C2 | 23 March 2022 |
| | | | | US | 2021275453 | A1 | 09 September 2021 |
| | | | | DK | 3372232 | T3 | 07 June 2021 |
| | | | | PL | 3372232 | T3 | 27 September 2021 |
| | | | | JPWO | 2017078008 | A1 | 09 August 2018 |
| | | | | JP | 6602886 | B2 | 06 November 2019 |
| | | | | RU | 2018119680 | A3 | 05 December 2019 |
| | | | | RU | 2018119680 | A | 11 December 2020 |
| | | | | MA | 43183 | A | 12 September 2018 |
| | | | | US | 2020197305 | A1 | 25 June 2020 |
| | | | | US | 11166914 | B2 | 09 November 2021 |
| | | | | WO | 2017078008 | A1 | 11 May 2017 |
| | | | | RU | 2020137384 | A | 03 December 2020 |
| | | | | RU | 2020137384 | A3 | 31 March 2021 |
| | | | | RU | 2761620 | C2 | 13 December 2021 |
| | | | | KR | 20180054873 | A | 24 May 2018 |
| | | | | KR | 102084340 | B1 | 03 March 2020 |
| | | | | ES | 2869283 | T3 | 25 October 2021 |
| | | | | EP | 3372232 | A1 | 12 September 2018 |
| | | | | EP | 3372232 | A4 | 14 November 2018 |
| | | | | EP | 3372232 | B1 | 14 April 2021 |
| CN | 106474067 | A | 08 March 2017 | None | | | |
| WO | 2005021012 | A1 | 10 March 2005 | JPWO | 2005021012 | A1 | 26 October 2006 |
| US | 2005249795 | A1 | 10 November 2005 | WO | 2004017944 | A1 | 04 March 2004 |
| | | | | AU | 2003268087 | A1 | 11 March 2004 |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 202210300041
- CN 106474067 B
- CN 102784107 B
- CN 102846547 B
- CN 111035616 A
- CN 111437259 A
- CN 108348538 A
- CN 111632030 A

**Non-patent literature cited in the description**

- Remington's Pharmaceutical Sciences. Pennsylvania: Mack Publishing Company, 1995